# EUROPEAN PATENT APPLICATION

(11) **EP 3 769 752 A1**
(43) Date of publication of application: **27.01.2021**
(21) Application number: 20187572.1
(22) Date of filing: 24.07.2020
(51) Int. Cl.: A61K 9/127, A61K 31/352, A61P 23/00

(54) **CANNABINOID-CONTAINING COMPOSITIONS IN THE FORM OF SPHERES OR SPHERE-LIKE PARTICLES, METHODS FOR THEIR PREPARATION, AND THERAPEUTIC APPLICATIONS**

(30) Priority: 26.07.2019 US 201962878994 P; 30.06.2020 US 202016917637
(71) Applicant: Landsteiner Scientific S.A. de C.V., 01900 Mexico City (MX)
(72) Inventor: GRANADOS CERVERA, Miguel Antonio, 1900 MEXICO CITY (MX); RIVERO GONZALEZ, Jose Angel, 50455 ATLACOMULCO (MX); MEJIA OLVERA, Eida Elena, 52160 METEPEC (MX); CARRANZA RUIZ, Martha Alicia, 45030 ZAPOPAN (MX); TELLEZ GARCIA, Juan Manuel, 50200 TOLUCA (MX); GAMARRA MORALES, Sarahi, 50200 TOLUCA (MX); KURI BRENA ROMERO DE TERREROS, Francisco, 01900 Mexico City (MX); MARIN CHINAS, Eric, 01900 Mexico City (MX); ARANDA LARA, Liliana, 01900 Mexico City (MX); MORALES AVILA, Enrique, 01900 Mexico City (MX)
(74) Representative: Boult Wade Tennant LLP

(57) **Abstract**

Pharmaceutical compositions and or formulations are in the form of spheres, or sphere-like particles. The pharmaceutical compositions include at least one isolated Cannabinoid or mixtures of at least two isolated Cannabinoid-elements obtained from raw or pre-treated vegetable material of Cannabis spp. (i.e. any species of Cannabis) or any synthetic or any semi-synthetic cannabinoid-mimicking compounds, that are formulated in encapsulated forms. Different routes of administration and desired therapeutic effects can be achieved with variations of the spheres and sphere-like particles.

## Description

### Field of Disclosure

The present application refers in general terms to pharmaceutical compositions and or formulations in the form of spheres, or sphere-like particles also referred as CannSpheres® along the specification of the present application, methods for preparing those compositions and or formulations and therapeutic applications thereof, said compositions comprising at least one isolated Cannabinoid or mixtures of at least two isolated Cannabinoid-elements obtained from raw or pre-treated vegetable material of *Cannabis spp.* (*i.e.* any species of Cannabis) or any synthetic or any semi-synthetic cannabinoid-mimicking compounds, that are formulated in encapsulated forms of very particular technical character.

### Background

Cannabis formulation technologies still face important challenges in terms of efficiency and efficacy. Technological challenges still remain grossly due to legal aspects related *Cannabis spp,* which impacts in the commercial demand for Cannabis-derived products. This fact is even more notorious when it comes to the pursuit of medicinal objectives and therapeutic level results in the use of these Cannabinoids as they require to go through sanitary controls unlike recreations Cannabis.

Also, medical use of Cannabinoids is of course subject to similar levels of care about purity, dose, toxicology, side effects, etc., as the rest of the known chemical and biotechnological therapeutic agents. However, a very sensitive aspect of utilizing Cannabinoids in a medical grade formulation is the fact that the natural origin of cannabinoids makes it inherently difficult to standardize purities, doses, and eliminate potential toxic or psico-active components that can be found in the raw vegetable material. Some of the above challenges are also true for synthetic and semi-synthetic components mimicking the actions of naturally-occurring cannabinoids.

The above should be added the fact that transportation of therapeutic agents through the intricate and variable systems of cell membranes to effectively reach a medical grade therapeutic effect require the provision of advanced pharmaceutical formulations that can effectively deliver Cannabinoids into their specific sites of action with acceptable bioavailability. Needless to say that such a delivery, besides being effective on the bioavailability, should be also efficient to avoid any undesirable secondary effect in the patient, and also avoiding rising the costs of manufacturing these types of pharmaceutical compositions in and industrial scale due to the imprecise and or uncontrolled process parameters.

### Brief description of the Drawings

Figure 1 is a schematic representation of some of the sphere embodiments of the pharmaceutical composition of the present application.
Figure 2. shows scanning electron microscopy images of the pharmaceutical composition obtained in example 2.c indicating the particle sizes and uniform distribution of the compositions. The arrows in Figures b) and c) show the formation of the lipid bilayer, showing the formation of liposomal structures.
Figure 3 is a size-distribution graph showing the improved distribution achieved for the pharmaceutical composition of example 2.c.
Figure 4 is a flow chart representing some of the embodiments of the process carried out for the preparation of the pharmaceutical compositions.
Figure 5 is a graphic representation of one of the embodiments of the process for preparing a pharmaceutical composition showing an exemplary arrangement the technique utilized.

### Detailed Description

An attempt to construct a delivery system for cannabis compounds that utilizes a multilayered lipid vesicle can be found in the US Patent application No. US 2018/0360757. This document aims to provide a system for the dermatological administration of Cannabinoids, which incorporates a controlled delivery matrix, and thus bases the existence of such a very complex multilayered vesicles into the difficulty of passing Cannabis compounds through the skin, while at the same highlighting the disadvantages of other administration routes for Cannabinoids, such as oral routes. Also, it is important to note that the particle sizes of the vesicles described therein render them to be catalogued as large molecules in the field of liposomes.

On the other hand, the document published as WO 01/03668 from The Minister of National Defense of her Majesty's Canadian Government, explored the formulation of liposome-encapsulated cannabinoids, specifically formulated for pulmonary administration, highlighting the problems of other routes of administration in terms of dosing and delivery of the active components of Cannabis.

On its end, WO 2016/010840 from Novartis AG describes an encapsulation technology that is specific for encapsulating DNA or RNA molecules based on the discovery that a combination of lipid streams and nucleic acid streams, at a specific linear velocity and with a specific concentration of residual organic solvent can efficiently encapsulate those specific types of molecules (*see 0023 and claims of* WO 2016/010840*).*

US20080188675A1 from SIRNA THERAPEUTICS INC, provides a serum-stable formulated molecular composition (e.g., comprising a biologically active molecules such as polynucleotides including siNA, antisense, aptamer, decoy, ribozyme, 2-5A, triplex forming oligonucleotide, or other nucleic acid molecules) in which the biologically active molecule is encapsulated in a lipid bilayer and is protected from degradation (for example, where the composition adopts a lamellar structure). Additionally, the formulated particles formed are preferably neutral or negatively-charged at physiological pH. Nevertheless, this application does not teach or suggest that other drugs or derivates of active pharmaceutical ingredients could be encapsulated in the composition.

WO2002072068A2 from the CANCER RESEARCH CAMPAIGN TECHNOLOGY LIMITED, describes in general terms lipid compositions comprising a cationic lipid fraction (A) and a non-ionic lipid fraction. Furthermore, the embodiments described therein pertains to the field of certain lipids and lipid compositions, liposomes and lipoplexes formed therefrom, methods for their synthesis and preparation, compositions and medicaments comprising such liposomes and lipoplexes, and methods of cellular delivery, transfection, and medical treatment employing such liposomes and lipoplexes.

CA2970651A1 from OJAI ENERGETICS PBC describes a composition comprising a plurality of microcapsules, wherein an individual microcapsule of the plurality comprises (a) at least one cannabinoid compound and (b) at least one terpene compound present in an amount of at least about one microgram, said microcapsules are characterized by providing a sigmoidal release profile of the at least one cannabinoid compound.

The above documents confirm that the release of therapeutic substances, including Cannabinoids, is unpredictable and will vary depending on the nature of the encapsulation materials utilized, the hydrophilic/lipophilic character of such materials and therapeutic substances as well as on the methods of preparation. Also, the specificity of the encapsulation technologies depends on other various factors, such as the materials to be encapsulated, the selected routes of administration, dosing, etc.

There is therefore a need for pharmaceutical formulations and methods for their preparation that specifically contain isolated Cannabinoids in a medical grade pharmaceutical presentation that can assure an effective delivery of the isolated Cannabinoids of mixtures thereof in a precise metered dose and bioavailability of the desired isolated Cannabinoids or mixtures thereof to be administrated for therapeutic, rehabilitator or preventive goals; and that eliminates barriers for patient compliance for side effects due to *Cannabis* spp.-derived impurities, and complex and uncomfortable routes of administration.

The present application is directed to provide novel pharmaceutical compositions which are formulated as colloidal particles in which an active substance, particularly, a Cannabinoid compound or mixtures of at least two Cannabinoid compounds can be encapsulated, adsorbed, absorbed or incorporated in any other adequate manner into such pharmaceutical compositions; and that are adequate and effective for the treatment of the symptoms and as therapeutic and preventing agents *per se* over a wide range of conditions, from treating for instance muscle pain for athletes in one side of the spectrum, and until treating cancer on the other side of the spectrum.

The pharmaceutical compositions of the present application offer significant advantages in terms of Cannabinoid delivery due to its special nano spherical-type arrangement and the components that conform such compositions, thus overcoming the technical challenges associated to the ionic nature of Cannabinoids, its purity, and the control of its lateral and side effects such as toxicology and psychotic effects.

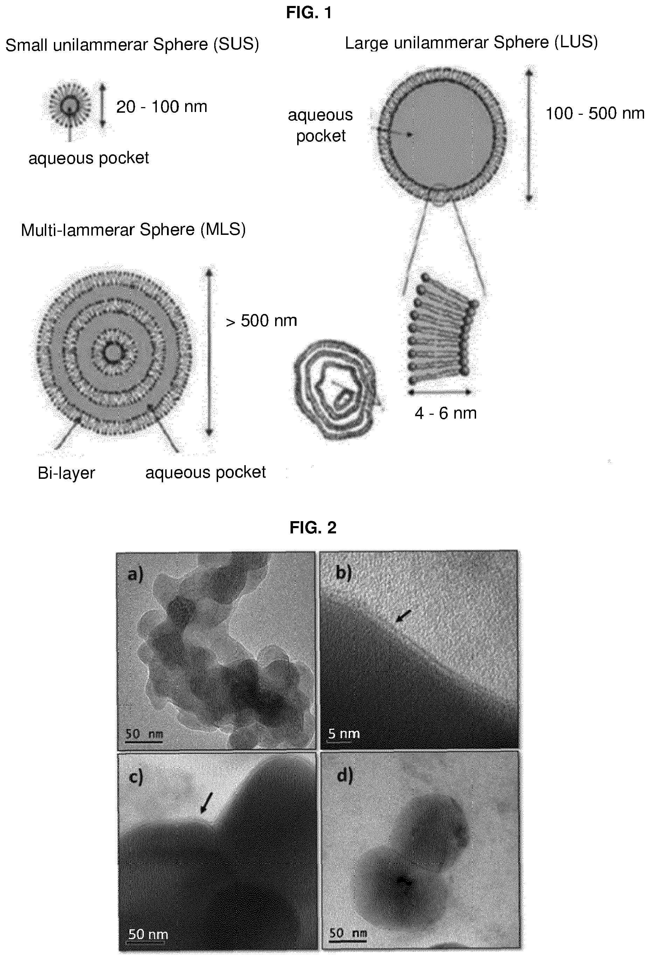

In some aspects, the compositions of the present application are formed in its most general description at least by the following elements: i) optionally, in its outermost layer, by a Fab (antigen-binding fragment) for either absorbing or adsorbing or securing to the system in any other effective way, a selective targeting molecule which may be or incorporate or otherwise carry a cannabinoid or a mixture of cannabinoids, or another cannabinoid-receptor agonist compound ii) a pharmaceutically acceptable polymer coating which may also incorporate or otherwise carry a cannabinoid or a mixture of cannabinoids, iii) at least one lipid component optionally in the form of a single or multiple membrane which may incorporate or otherwise carry a cannabinoid or a mixture of cannabinoids, iv) an internal lipid-aqueous interface which may incorporate or otherwise carry a cannabinoid or a mixture of cannabinoids, and v) a nucleus, optionally containing the cannabinoid compound. Also, a series of solvents, buffers and water are utilized as adjuvants for the formulation of the above mentioned compositions. A schematic representation of the different examples of the sphere or sphere-like configuration of the compositions of the present application can be seen in Figure 1 and Figure 2, described in detail in the following section of this application.

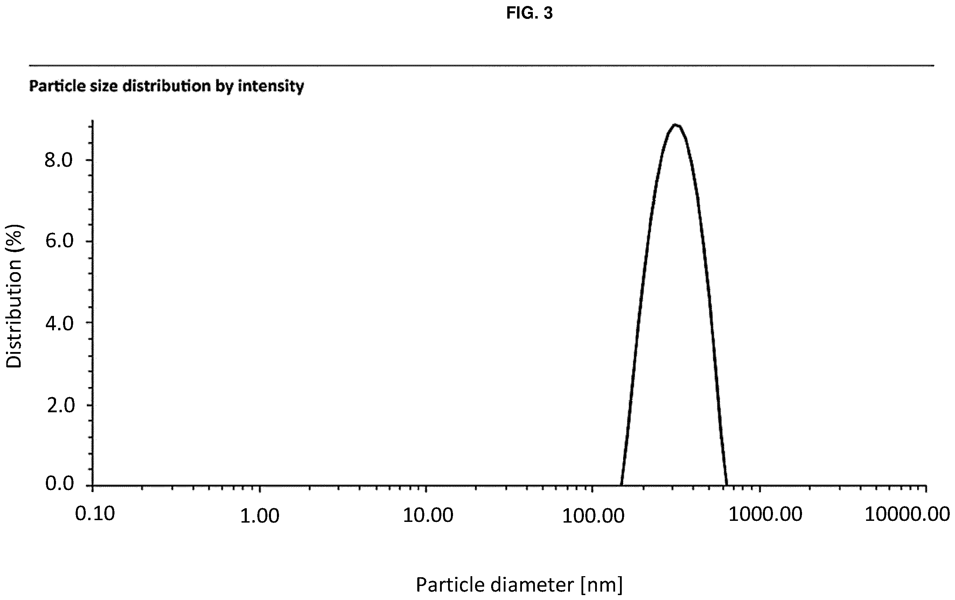

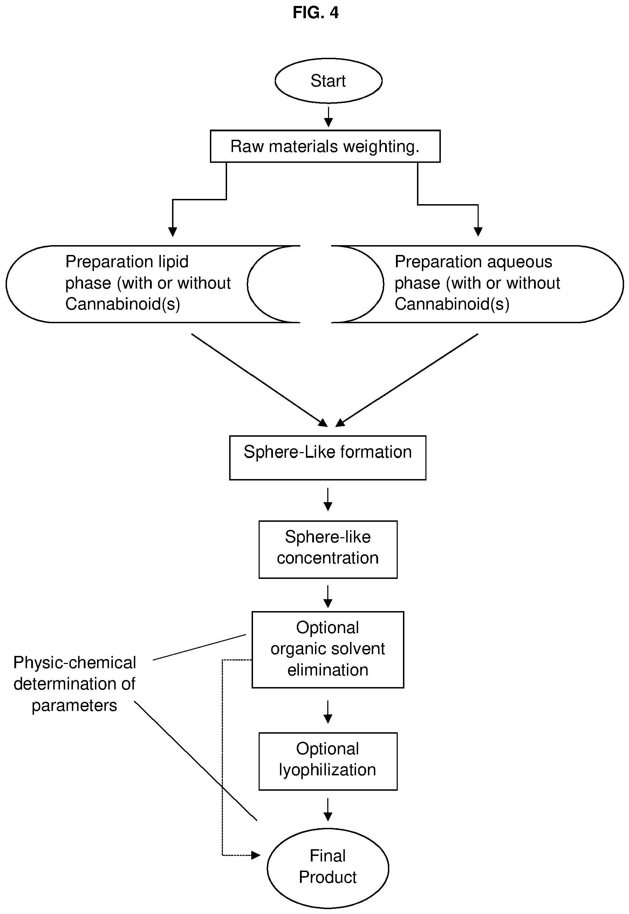

Also, in its most general description, the processes for preparing these nano spherical composition systems are based upon the use of microfluid-technologies that demonstrated for the particular systems and under the particular conditions defined in the present application, a high efficacy of near 100% of encapsulation, a monodipersity/dispersity value of the polymerized chains in the final products of below 5%, a significant ability in controlling the particle size and the possibility of obtaining uniform and homogenous batches for the final products with a polydispersion value of below 20%. A general scheme of preparation can be found in Figures 3 and 4, described in detail herein.

*In vitro* tests were performed to demonstrate the ability of the pharmaceutical compositions of the present applications to effectively cross the complex and intricate membrane systems of cells.

Also, two clinical studies for assessing the effect of the pharmaceutical compositions of the present application were performed to measure the effect of the CBDs and THCs systems as therapeutic agents. In the first one of these studies, 32 patients suffering from different stages of gastrointestinal cancers were administered with the pharmaceutical compositions of the present application, resulting in at least 10% of improved tumoral reduction response in comparison with chemotherapy alone. The second clinical study was performed on patients having irritable bowel syndrome (IBS) in which 50 patients were administered with the pharmaceutical composition systems of the present application, showing significant improvements on the inflammatory pattern, oxidative stress and plasmatic cytosine levels of their IBS.

Results of both *in vitro* and *in vivo* tests suggest that the sphere or sphere-like pharmaceutical compositions of the present application are efficient vehicles for delivering isolated cannabinoid compounds or mixtures of at least two of such compounds with a good bioavailability and low to zero psychoactive or undesired toxic effects. These results also confirm that the process of preparation described in the present application (which as it is well know from the art, determines performance of final products) for the sphere or sphere-like pharmaceutical compositions of the present application is also unique as it allows obtaining the advantageous final products of the present application.

### Pharmaceutical compositions.

For the purposes of the present application, the term Cannabinoid or Cannabinoids as used herein means any individually identifiable compound or substance that is either synthetic or artificially produced, or that can be obtained by any mean from any part of any species of a Cannabis vegetal material, including, hybrids, genetically modified species, and any other variant of plant artificially or naturally occurring that may contain cannabinoids, such as, without limitation, roots, leaves, thallus, stems, flowers, buds, nodes, etc.

Also encompassed within the scope of the present application are Cannabinoids as defined above, that could be obtained from resins, pollen, grains, seeds and any other product or precursors of any species of a Cannabis vegetal material, including, hybrids, genetically modified species, and any other variant of plant artificially or naturally occurring.

The pharmaceutical compositions or formulations of the present application can include therefore, at least one, and or any combination of two or more Cannabinoids selected from the group comprising: *Cannabichromenes, including:* Cannabichromene (CBC), Cannabichromenic acid (CBCA), Cannabichromevarin (CBCV), Cannabichromevarinic acid (CBCVA), *Cannabicyclols, including:* Cannabicyclol (CBL), Cannabicyclolic acid (CBLA), Cannabicyclovarin (CBLV), *Cannabidiols, including:* Cannabidiol (CBD), Cannabidiol monomethylether (CBDM), Cannabidiolic acid (CBDA), Cannabidiorcol (CBD-C1), Cannabidivarin (CBDV), Cannabidivarinic Acid (CBDVA), *Cannabielsoins, including:* Cannabielsoic acid B (CBEA-B), Cannabielsoin (CBE), Cannabielsoin acid A (CBEA-A), *Cannabigerols:* Cannabigerol (CBG), Cannabigerol monomethylether (CBGM), Cannabigerolic acid (CBGA), Cannabigerolic acid monomethylether (CBGAM), Cannabigerovarin (CBGV), Cannabigerovarinic acid (CBGVA), *Cannabinols and cannabinodiols, including:* Cannabinodiol (CBND), Cannabinodivarin (CBVD), Cannabinol (CBN), Cannabinol methylether (CBNM), Cannabinol-C2 (CBN-C2), Cannabinol-C4 (CBN-C4), Cannabinolic acid (CBNA), Cannabiorcool (CBN-C1), Cannabivarin (CBV), *Cannabitriols, including:* 10-Ethoxy-9-hydroxy-delta-6a-tetrahydrocannabinol, 8,9-Dihydroxy-delta-6a-tetrahydrocannabinol, Cannabitriol (CBT), Cannabitriolvarin (CBTV), *Delta-8-tetrahydrocannabinols, including:* Delta-8-tetrahydrocannabinol (Δ8-THC), Delta-8-tetrahydrocannabinolic acid (Δ8-THCA), Delta-9-tetrahydrocannabinols, Delta-9-tetrahydrocannabinol (THC), Delta-9-tetrahydrocannabinol-C4 (THC-C4), Delta-9-tetrahydrocannabinolic acid A (THCA-A), Delta-9-tetrahydrocannabinolic acid B (THCA-B), Delta-9-tetrahydrocannabinolic acid-C4 (THCA-C4), Delta-9-tetrahydrocannabiorcol (THC-C1), Delta-9-tetrahydrocannabiorcolic acid (THCA-C1), Delta-9-tetrahydrocannabivarin (THCV), Delta-9-tetrahydrocannabivarinic acid (THCVA). 10-Oxo-delta-6a-tetrahydrocannabinol (OTHC), Cannabichromanon (CBCF), Cannabifuran (CBF), Cannabiglendol, Cannabiripsol (CBR), Cannbicitran (CBT), Dehydrocannabifuran (DCBF), Delta-9-cis-tetrahydrocannabinol (cis-THC), Tryhydroxy-delta-9-tetrahydrocannabinol (triOH-THC), 3,4,5,6-Tetrahydro-7-hydroxy-alpha-alpha-2-trimethyl-9-n-propyl-2,6-methano-2H-1-benzoxocin-5-methanol (OH-iso-HHCV), and any salt, enantiomer, isomer, aptamer, free acid or free base, and any mixture of any of those salts, enantiomers, isomers, aptamers, free acids, or free bases thereof.

The concentration of the at least one Cannabinoid, or the any possible mixture of at least two Cannabinoids can range from about 0.00795 mM to about 25 mM in the final pharmaceutical product. The term about means a range of (+/-) 15% of the accompanying value.

The Fab (antigen-binding Fragment) that can be utilized by the present application can be any antigen binding region (Fab, Fab', F (ab') 2 and Fv) even Fabs such as ScFv, ds-Fv, ds-ScFv, diabody, triabody, tetrabody, Bis-scFv, minibodies, Fab2, Fab3, etc.), or any other Fab which allows to maintain the function of the active orientation, of the CannSpheres towards specific antigens.

The lipidic portion of the pharmaceutical compositions of the present disclosure can be constructed utilizing at least one lipid that ranges from any linear or any branched short chains of saturated or unsaturated CH₂ groups linked by carbon-carbon bonds with at least one terminal carboxylic acid group, to any linear or any branched long chains of saturated or unsaturated CH₂ groups linked by carbon-carbon bonds with at least one terminal carboxylic acid group. These lipids can be saturated, non-saturated or partially saturated in terms of the amount of hydrogen atoms present or absent attached to the Carbon atoms of their structures. Also, the technology of the present application can be put into practice with cationic, neutral, anionic, or mixed character (amphiphilic) of the aforementioned categories of lipids.

A non-limitative exemplary list of at least one fatty acid and the those fatty acids conforming the mixture of at least two fatty acids that can be included in the different cases of the present application is: Acetic 2:0, Acetonic 2-OH, 2-Me 3:0; Acrylic 2e-3:1; Adipic 6:0 di-acid; Adrenic 7c10c13c16c-22:4; Agonandoic 9a11t-18:2; Agonandric 8-OH, 9c11t-18:2; Ajenoic 3c5c7c9c11e-12:5; Alchornoic cis-14,15-ep 11c-20:1; Alepraic/Alepramic 3-Cp 3:0; Aleprestic 5-Cp 5:0; Alepric 9-Cp 9:0; Aleprolic 1-Cp 1:0; Aleprylic 7-Cp 7:0; Aleuritic 9,10,16-triOH 16:0; Aleutiric 9,10,18-triOH 18:0; Alvaradoic 6a17e-18:2; Alvaradonic 8a17e-18:2; Ambrettolic 16-OH, 7c-16:1; Anacyclic 2t4t8a10a-14:4; Angelic (2Z)-2-Me 4:1; Anteisoheptadecanoic 14Me 16:0; Anteisononadecanoic 16Me 18:0; Anteisopentadecanoic 12Me 14:0; Anteisotridecanoic 10Me 12:0; Arachidic 20:0; Arachidonic 5c8c11c14c-20:4; Argenonic 6-OH 6-Me, 9-oxo-28:0; Asclepic 11c-18:1; Athanacalvic 9-OH 9t16c12a14a-18:4; Auricolic 14-OH 11c17c-20:2; Avenoleic 15(R)-OH 9c12c-18:2; Axillarenic (Axillaric) 11,13-di OH, 9c-24:1; Azelaic 9:0 di-acid; Behenic 22:0; Behenolic 13a-22:0; Bishomocolumbinic 7c11c14t-20:3; Bishomopinolenic 7c11c14c-20:3; Bolekic 9a11a13c-18:3; Bosseopentaenoic 5c8c10t12t14c-20:5; Brassidic 13t-22:1 (*trans* form of erucic acid); Brassylic 13:0 di-acid; Buiolic 11-OH 16:0; Butolic 6-OH, 14:0; Butyric 4:0; Caleic 3t9t12c-18:3; Calendic (α) 8t10t12c-18:3; Calendic (β) 8t10t12t-18:3 (*trans* form of α-calendic acid); Callosobruchusic 3,7-di-Me,2c-8:1 di-acid; Capric 10:0; Caproic 6:0; Caproleic 9c-10:1; Caprylic 8:0; Carboceric 27:0; Cascarillic 3,4-Mt-10:0; Catalpic 9t11t13c-18:3; Cerebronic 2-OH 24:0; Ceroplastic 35:0; Cerotic 26:0; Cetelaidic 11t-22:1; Cetoleic 11c-22:1; Chaulmoogric 13-Cp 13:0; Chrysobalanic 4-oxo 9c11t13t15c-18:4; Cilienic 6,11c-18:2; Citraconic (Z)-2-Me 4:1 di-acid; Civetic 8t-17:1 di-acid; CLA Conjugated 18:2 isomers; Clupanodonic 4c8c12c15c19c-20:5c; Colneleic 9-oxa-8t10t12c-18:3; Colnelenic 9-oxa-8t10t12c15c-18:4; Columbinic 5t9c12c-18:3; Coniferonic 5c9c12c15c-18:4; Convolvulinolic 3,12-diOH-16:0 or Convolvulinolic 11-OH 15:0 or Convolvulinolic 11-OH 14:0; Coriolic 13-OH 9c11t-18:2; Coronaric cis-9,10-ep 12c-18:1; Crepenynic (Crepeninic) 9c12a-18:2; Crotonic 2t-4:1; Dehydrocrepenyic 9c12a14c-18:3; Demospongic C24-C34 5c9c-diene acids; Dendrotrifidic 16-OH, 9c12a14a17e-18:4; Dendryphiellic 6-Me 2c4c-8:2; Densipolic 12(R)-OH 9c15c-18:2; Denticetic 5c-12:1; DHA 4c7c10c13c16c19c-22:6; Dicramin 6a9c12c15c-18:4; Dihomogammalinolenic 8c11c14c-20:3; Dihomolinoleic 11c14c-20:2; Dihomolinolenic 11c14c17c-20:3; Dihomo Mead's acid 7c10c13c-22:3; Dihomopinolenic 7c11c14c-20:3; Dihomotaxoleic 7c11c-20:2; Dihydrofulgidic 9S,12S,13S-tri-OH 10t-18:1; Dihydromalvalic 8,9-P-18:0; Dihydromalyngic 9S,12R,13S-tri-OH 10t-18:1; Dihydroxystearic 9,10-diOH 18:0; α-Dimorphecolic 9-OH,10t12c-18:2; β-Dimorphecolic 9-OH,10t12t-18:2; DPA 7c10c13c16c19c-22:5; Elaidic 9t-18:1 (*Trans* isomer of oleic acid Elaidolinolenic); EPA 5c8c11c14c17c-20:5; Eleostearic (α) 9c11t13t-18:3; Eleostearic (β) 9t11t13t-18:3; Enanthic (enanthoic) 7:0; Ephedrenic 5c11c-18:2; Equisetolic 30:0 di-acid; Eranthic 5c13c16c-22:3; Erucic 13c-22:1; D-Erythronic 2R,3R,4 tri-OH 4:0; L-Erythronic 2S,3S,4 tri-OH 4:0; Exocarpic 9a11a13t-18:3; Farnesenic 3,7,11-tri-Me 2,6,10-15:3; Fomentic 2-Me 3,3-di-18:0 4:0 di-acid; Fuconic 2,3,4,5-tetraOH 6:0; Fulgidic 9R,12R,13R tri-OH 10t,15c18:2; Fumaric 2t-4:1 di-acid; Furocarpic 9,12-ep, 9t12t-18:2; Gadelaidic 9t-20:1 (*trans* form of gadoleic acid); Gadoleic 9c-20:1; Gaidic 2t-16:1; Geddic 34:0; Geranic 3,7-di-Me 2t6e-8:2; Gheddic 34:0, GLA 6c9c12c-18:3; Glutamic 2-NH2 5:0 di-acid; Glutaric 5:0 di-acid; Glycolic 2-OH 2:0; Gondoic 11c-20:1; Gondoleic 9c-20:1; Gorlic 13-Cp 6c-13:1; Goshuyic 5c8c-18:2; Helenynolic 9-OH 10t12a-18:2; Hiragonic 6c10c14c-16:3; Homotoluic PhCH2CH2COOH; Hormelic 15-Cp 15:1; Hydnocarpic 11-Cp 11:0; Hydrosorbic 3t-16:1 di-acid ; 11-Hydroxyceromelissic 11-OH 33:0 Hydroxynervonic 2-OH 15c-24:1; Hyenic 25:0; Ipurolic 3,11-diOH 14:0; Isanic 9a11a17e-18:3; Isanolic 8-OH 9a11a17e-18:3; Isoarachidic 18-Me 19:0 ; Isobutyric 2-Me 3:0; Isocapric 8-Me 9:0; Isocaproic 4-Me 5:0 ; Isocerotic 24-Me 25:0 ; Isocrotonic 2c-4:1; Isogorlic 4c,2-Cp-18:2; Isoheptadecanoic 15-Me-16:0; Isolauric 10-Me-11:0; Isoleucic 2R-Me, 3R-OH 6:0; Isomargaric 15-Me 16:0; Isomontanic 26-Me-27:0; Isomycomycin 3c5c7a9a11a-13:5; Isomyristic 12-Me 13:0; Isononadecanoic 17-Me 18:0; Isooctadecanoic i-18:0; 16Me-17:0; Isooleic 10c-18:0; Isopalmitic 14-Me 15:0; Isopentadecanoic 13-Me 14:0; Isoricinoleic 9-OH,12c-18:1; Isostearic 16-Me 17:0; Isotridecanoic 11-Me 12:0; Isovaleric 3-Me 4:0; Ixoric 8c10c12c14t-18:4; Jacaric 8c10t12c-18:3; Jalapinolic 11-OH 16:0; Japanic 21:0 di-acid; Juniperic 16-OH 16:0; Juniperinic 16-OH 16:0; Juniperonic 5c11c14c17c-20:4; Kamlolenic (α) 18-OH 9c11t13t-18:3; Kamlolenic (β) 18-OH 9t11t13t-18:3; Kerrolic 4-OH 16:0; Keteleeronic 5c11c-20:2; Labellenic 5e,6e-18:2; Lacceric 32:0; Lactarinic 6-oxo 18:0; Laetisaric 8-OH, 9c12c-18:2; Lanoceric di-OH 30:0; Lamenallenic 5c,6c-18:2; Lactobacillic 11,12-Mt 18:0; Lauric 12:0; Lauroleic 9c-12:1; Lesquerolic 14-OH 11c-20:1; Leucic 2-OH, 4-Me 5:0; Levulinic 4-oxo-5:0; Licanic (α) 4-oxo 9c11t13t-18:3; Licanic (β) 4-oxo 9t11t13t-18:3 (Trans-form of α-licanic acid); Lignoceric 24:0; Linderic 4c-12:1; Linelaidic 9t12t-18:2; Linoleic 9c12c-18:2; Linolenelaidic 9t12t15t-18:3; Linolenic 9c12c15c-18:3; Lumequic 21c-30:1; Linusic 9,10,12,13,15,16-hexaOH 18:0; Lycaonic 12-oxo-18:0; Lycopodic 11t-16:1; Maleic 2c-4:1 di-acid; DL-Malic 2-OH 4:0 di-acid; Malonic 3:0 di-acid; Malvalic 8,9-P 8c-18:1; Malyngic 9S,12R,13S tri-OH 10t,15c-18:2; Manaoic/manoaic 11-Cp 6c-11:0; Mangold's acid 9t11t-18:2; Margaric 17:0; Matricaria acid 2t4a6a8t-10:4; Z-E- Matricaric 2c4a6a8t-10:4; Mead's acid 5c8c11c-20:3; Megatomic (megatomoic) 3t5c-14:2; Mesaconic (E)-2-Me-4:1 di-acid; Melissic 30:0; Mikusch's acid 10t12t-18:2; Minquartynoic 17-OH-9a11a13a15a-18:4; Montanic 28:0; Mycinonic 4-Me, 5-OH 2t-7:1; Mycoceranic 2,4,6-tri-Me 26:0; Mycolic RCHOHCH(R')COOH; Mycolipenic 2,4,6-tri-Me-2t-24:1; Mycomycin 3t5c7e8e10a12a-13:6; Myristelaidic 9t-14:1 (Trans form of myristoleic acid) Myristic 14:0; Myristoleic 9c-14:1; Nemotinic 4-OH, 5e6e8a10a-11:4; Nerolic 3,7-di-Me 2c6e-8:2; Nervonic 15c-24:1; Nisinic 24:6, all- cis-6,9,12,15,18,21; Norlinoleic 8c11c-17:2; Norlinolenic 8c11c14c-17:3 Libiatae Obtusilic 4c-14:1; Oleic 9c-18:1; Oncobic 15-Cp 8c-15:1; Oxalic 2:0 di-acid; Palmitelaidic 9t-16:1 (trans form of petroselinic acid); Palmitic 16:0; Palmitoleic 9c-16:1; Parinaric (α) 9c11t13t15c-18:4; Parinarium laurinum Parinaric (β) 9t11t13t15t-18:4 (*trans* form of a-parinaric acid); Paullinic 13c-20:1; Pelargonic 9:0; Palmitvaccenic 11c-16:1; Petroselaidic 6t-18:1 (Trans form of petroselinic acid); Petroselinic 6c-18:1; Phellogenic 22:0 di-acid; Phellonic 22-OH 22:0; Phlomic 7e,8e-20:2; Phthioic 3,13,19-tri-Me 23:0; Physeteric 5c-14:1; Phytanic 3,7,11,15-tetra-Me 16:0; Phytenoic 3,7,11,15-tetra-Me 2e-16:1; Pimelic 7:0 di-acid; Pinolenic 5c9c12c-18:3; Pivalic 2,2-di-Me 3:0; Podocarpic 5c11c14c-20:3; Pristanic 2,6,10,14-tetra-Me 15:0; Propioic (propynoic) 2a-3:0; Pseudoeleostearic 10t12t14t-18:3; Psyllic 33:0; Punicic 9c11t13c-18:3; Pyrulic 8a10t-17:2; Rhodinic 3,7-di-Me,6e-8:1; Ricinelaidic 12-OH 9t-18:1 (trans form of ricinoleic acid); Ricinenic 9c,11c-18:2 Ricinoleic 12-OH 9c-18:1; Rosilic 10-OH 18:0; Roughanic acid 16:3(n-3); Rumenic 9c11t-18:2; Sabinic 12-OH 12:0; Sapienic 6c-16:1; Sarcinic 12Me-14:0; Sativic (santivinic) 9,10,12,13-tetraOH 18:0; Scoliodonic 24:5; Sebacic 10:0 di-acid; Sebaleic 5c,8c-18:2; Shibic 26:5; Sorbic 2t4t-6:2 di-acid; Stearic 18:0; Stearidonic 6c9c12c15c-18:4; Stearolic 9a-18:1; Sterculic 9,10-P 9c-18:1; Sterculynic 9,10-Mt 9c17a-18:2; Stillingic 2c4t-10:2; Suberic 8:0 di-acid; Succinic 4:0 di-acid; Tariric 6a-18:1; Tartaric 2,3-diOH 4:0 di-acid; Taxoleic 5c9c-18:2; Thalictric 5t-18:1; Thaspic 16:0 di-acid; (+/-) Threonic 2,3,4 tri-OH 4:0; D-Threonic 2R,3S,4 tri-OH 4:0; L-Threonic 2S,3R,4 tri-OH 4:0; Threonine 2-NH2, 3-OH 4:0; Thynnic 26:6 (n-3); Tiglic (2E)-2-Me 4:1; Traumatic 2t-20:1 di-acid; Tsuduic 4c-14:1; Tsuzuic 4c-14:1; Tuberculostearic 10-Me 18:0; Undecylenic 10e-11:1; Ursolic 30-OH-30:0; Ustilic 15,16-diOH 16:0; Ustilic B 2,15,16-triOH 16:0; Valeric 5:0; Valproic 2-Propyl 5:0; 9,10,12,13-tetraOH-22:0; Vernolic cis-12,13-ep, 9c-18:1; Wyeronic acid 4,7-ep, 8-oxo, 2t9a11c-14:5; Ximenic 17c-26:1; Ximenynic 9a11t-18:2; Ximenynolic 8-OH, 9a11t-18:2; Xionenynic 8a10c-18:2; and any isomers, salts, free salts, derived acids and derives bases thereof.

Also, the lipid can be a phospholipid compound, selected from any individual phospholipid belonging to the following groups: Phosphatidylcholine Phosphatidic acid; Lysophosphatidylcholine; Phosphatidylethanolamine; Phosphatidylglycerol; Phosphatidylserine; PEG phospholipid (mPEG-phospholipid, polyglycerin-phospholipid, funcitionalized-phospholipid, terminal activated-phospholipid); including but not limited to Phosphatidic acid (phosphatidate) (PA); Phosphatidylethanolamine (cephalin) (PE); Phosphatidylcholine (lecithin) (PC); Phosphatidylserine (PS); Phosphatidylinositol (PI); Phosphatidylinositol phosphate (PIP); Phosphatidylinositol bisphosphate (PIP2); Phosphatidylinositol trisphosphate (PIP3); and any aptamers, isomers, salts, free salts, free bases, free acids, derived acids and derived bases thereof. Phospholipids utilized to construct the composition of the present application can be PEGylated molecules or not.

The concentrations in which such at least one lipid can be present in the different examples of the compositions of the present application range from about 0.0007959 mM to about 200 mM for lipids, fatty acids or any of its derivatives; from about 0.00954077 mM to about 2,28977850mM for phospholipids or any of its derivatives.

The pharmaceutical compositions can further include Cholesterol ((3β)-cholest-5-en-3-ol, or (3S,8S,9S,10R,13R,14S,17R)-10,13-dimethyl-17-[(2R)-6-methylheptan-2-yl]-2,3,4,7,8,9,11,12,14,15,16,17-dodecahydro-1H-cyclopenta[a]phenanthren-3-ol) and Cholesterol molecules derivatives thereof in the form of salts or crystals, to reduce permeability of the membrane making the spheres' structure to become more rigid thus improving their stability in the presence of biologic fluids such as blood. These Cholesterol or cholesterol-type or cholesterol derived molecules or triglyceride molecules can be selected from the group comprising: 22(S)-Hydroxycholesterol; 25-Hydroxycholesterol; 5α-Cholest-7-en-3β-ol; 5α-Cholestan-3β-ol; 5α-Cholestan-3-one (amorph or crystalline); 5-α-Cholestane; 5β-Cholestan-3α-ol; 5-Cholesten-3β-ol-7-one, 7β-Hydroxycholesterol; Campesterole; Cholesta-3,5-diene; Cholestanol; Cholesterol 5α,6α-epoxide; Cholesterol 5β,6β-epoxide; Cholesterol-PEG 600; Cholesteryl acetate; Cholesteryl arachidonate; Cholesteryl behenate; Cholesteryl erucate; Cholesteryl linoleate; Cholesteryl linolelaidate; Cholesteryl n-valerate; Cholesteryl oleate; Cholesteryl palmitate; Cholesteryl palmitelaidate; Cholesteryl pelargonate; Cholesteryl phenylacetate; Cholesteryl (pyren-1-yl)hexanoate; Cholesteryl stearate; Ganoderic acid A; Glycocholic acid hydrate; Lanosterol; Sodium cholesteryl sulfate; Thiocholesterol, and any isomers, salts, free salts, derived acids and derives bases thereof.

Also, mono, bi, or triglyceride molecules, or any molecule being a tri-ester type molecule core consisting of a glycerol bound to one, two or three fatty acid molecules, including or not either one, or two, or three of their hydroxyl portions coupled to a fatty acid selected from the group comprising: Acetic 2:0, Acetonic 2-OH, 2-Me 3:0; Acrylic 2e-3:1; Adipic 6:0 di-acid; Adrenic 7c10c13c16c-22:4; Agonandoic 9a11t-18:2; Agonandric 8-OH, 9c11t-18:2; Ajenoic 3c5c7c9c11e-12:5; Alchornoic cis-14,15-ep 11c-20:1; Alepraic/Alepramic 3-Cp 3:0; Aleprestic 5-Cp 5:0; Alepric 9-Cp 9:0; Aleprolic 1-Cp 1:0; Aleprylic 7-Cp 7:0; Aleuritic 9,10,16-triOH 16:0; Aleutiric 9,10,18-triOH 18:0; Alvaradoic 6a17e-18:2; Alvaradonic 8a17e-18:2; Ambrettolic 16-OH, 7c-16:1; Anacyclic 2t4t8a10a-14:4; Angelic (2Z)-2-Me 4:1; Anteisoheptadecanoic 14Me 16:0; Anteisononadecanoic 16Me 18:0; Anteisopentadecanoic 12Me 14:0; Anteisotridecanoic 10Me 12:0; Arachidic 20:0; Arachidonic 5c8c11c14c-20:4; Argenonic 6-OH 6-Me, 9-oxo-28:0; Asclepic 11c-18:1; Athanacalvic 9-OH 9t16c12a14a-18:4; Auricolic 14-OH 11c17c-20:2; Avenoleic 15(R)-OH 9c12c-18:2; Axillarenic (Axillaric) 11,13-di OH, 9c-24:1; Azelaic 9:0 di-acid; Behenic 22:0; Behenolic 13a-22:0; Bishomocolumbinic 7c11c14t-20:3; Bishomopinolenic 7c11c14c-20:3; Bolekic 9a11a13c-18:3; Bosseopentaenoic 5c8c10t12t14c-20:5; Brassidic 13t-22:1 (*trans* form of erucic acid); Brassylic 13:0 di-acid; Buiolic 11-OH 16:0; Butolic 6-OH, 14:0; Butyric 4:0; Caleic 3t9t12c-18:3; Calendic (α) 8t10t12c-18:3; Calendic (β) 8t10t12t-18:3 (*trans* form of α-calendic acid); Callosobruchusic 3,7-di-Me,2c-8:1 di-acid; Capric 10:0; Caproic 6:0; Caproleic 9c-10:1; Caprylic 8:0; Carboceric 27:0; Cascarillic 3,4-Mt-10:0; Catalpic 9t11t13c-18:3; Cerebronic 2-OH 24:0; Ceroplastic 35:0; Cerotic 26:0; Cetelaidic 11t-22:1; Cetoleic 11c-22:1; Chaulmoogric 13-Cp 13:0; Chrysobalanic 4-oxo 9c11t13t15c-18:4; Cilienic 6,11c-18:2; Citraconic (Z)-2-Me 4:1 di-acid; Civetic 8t-17:1 di-acid; CLA Conjugated 18:2 isomers; Clupanodonic 4c8c12c15c19c-20:5c; Colneleic 9-oxa-8t10t12c-18:3; Colnelenic 9-oxa-8t10t12c15c-18:4; Columbinic 5t9c12c-18:3; Coniferonic 5c9c12c15c-18:4; Convolvulinolic 3,12-diOH-16:0 or Convolvulinolic 11-OH 15:0 or Convolvulinolic 11-OH 14:0; Coriolic 13-OH 9c11t-18:2; Coronaric cis-9,10-ep 12c-18:1; Crepenynic (Crepeninic) 9c12a-18:2; Crotonic 2t-4:1; Dehydrocrepenyic 9c12a14c-18:3; Demospongic C24-C34 5c9c-diene acids; Dendrotrifidic 16-OH, 9c12a14a17e-18:4; Dendryphiellic 6-Me 2c4c-8:2; Densipolic 12(R)-OH 9c15c-18:2; Denticetic 5c-12:1; DHA 4c7c10c13c16c19c-22:6; Dicramin 6a9c12c15c-18:4; Dihomogammalinolenic 8c11c14c-20:3; Dihomolinoleic 11c14c-20:2; Dihomolinolenic 11c14c17c-20:3; Dihomo Mead's acid 7c10c13c-22:3; Dihomopinolenic 7c11c14c-20:3; Dihomotaxoleic 7c11c-20:2; Dihydrofulgidic 9S,12S,13S-tri-OH 10t-18:1; Dihydromalvalic 8,9-P-18:0; Dihydromalyngic 9S,12R,13S-tri-OH 10t-18:1; Dihydroxystearic 9,10-diOH 18:0; α-Dimorphecolic 9-OH,10t12c-18:2; β -Dimorphecolic 9-OH,10t12t-18:2; DPA 7c10c13c16c19c-22:5; Elaidic 9t-18:1 (*Trans* isomer of oleic acid Elaidolinolenic); EPA 5c8c11c14c17c-20:5; Eleostearic (α) 9c11t13t-18:3; Eleostearic (β) 9t11t13t-18:3; Enanthic (enanthoic) 7:0; Ephedrenic 5c11c-18:2; Equisetolic 30:0 di-acid; Eranthic 5c13c16c-22:3; Erucic 13c-22:1; D-Erythronic 2R,3R,4 tri-OH 4:0; L-Erythronic 2S,3S,4 tri-OH 4:0; Exocarpic 9a11a13t-18:3; Farnesenic 3,7,11-tri-Me 2,6,10-15:3; Fomentic 2-Me 3,3-di-18:0 4:0 di-acid; Fuconic 2,3,4,5-tetraOH 6:0; Fulgidic 9R,12R,13R tri-OH 10t,15c18:2; Fumaric 2t-4:1 di-acid; Furocarpic 9,12-ep, 9t12t-18:2; Gadelaidic 9t-20:1 (*trans* form of gadoleic acid); Gadoleic 9c-20:1; Gaidic 2t-16:1; Geddic 34:0; Geranic 3,7-di-Me 2t6e-8:2; Gheddic 34:0, GLA 6c9c12c-18:3; Glutamic 2-NH2 5:0 di-acid; Glutaric 5:0 di-acid; Glycolic 2-OH 2:0; Gondoic 11c-20:1; Gondoleic 9c-20:1; Gorlic 13-Cp 6c-13:1; Goshuyic 5c8c-18:2; Helenynolic 9-OH 10t12a-18:2; Hiragonic 6c10c14c-16:3; Homotoluic PhCH2CH2COOH; Hormelic 15-Cp 15:1; Hydnocarpic 11-Cp 11:0; Hydrosorbic 3t-16:1 di-acid ; 11-Hydroxyceromelissic 11-OH 33:0 Hydroxynervonic 2-OH 15c-24:1; Hyenic 25:0; Ipurolic 3,11-diOH 14:0; Isanic 9a11a17e-18:3; Isanolic 8-OH 9a11a17e-18:3; Isoarachidic 18-Me 19:0 ; Isobutyric 2-Me 3:0; Isocapric 8-Me 9:0; Isocaproic 4-Me 5:0 ; Isocerotic 24-Me 25:0 ; Isocrotonic 2c-4:1; Isogorlic 4c,2-Cp-18:2; Isoheptadecanoic 15-Me-16:0; Isolauric 10-Me-11:0; Isoleucic 2R-Me, 3R-OH 6:0; Isomargaric 15-Me 16:0; Isomontanic 26-Me-27:0; Isomycomycin 3c5c7a9a11a-13:5; Isomyristic 12-Me 13:0; Isononadecanoic 17-Me 18:0; Isooctadecanoic i-18:0; 16Me-17:0; Isooleic 10c-18:0; Isopalmitic 14-Me 15:0; Isopentadecanoic 13-Me 14:0; Isoricinoleic 9-OH,12c-18:1; Isostearic 16-Me 17:0; Isotridecanoic 11-Me 12:0; Isovaleric 3-Me 4:0; Ixoric 8c10c12c14t-18:4; Jacaric 8c10t12c-18:3; Jalapinolic 11-OH 16:0; Japanic 21:0 di-acid; Juniperic 16-OH 16:0; Juniperinic 16-OH 16:0; Juniperonic 5c11c14c17c-20:4; Kamlolenic (α) 18-OH 9c11t13t-18:3; Kamlolenic (β) 18-OH 9t11t13t-18:3; Kerrolic 4-OH 16:0; Keteleeronic 5c11c-20:2; Labellenic 5e,6e-18:2; Lacceric 32:0; Lactarinic 6-oxo 18:0; Laetisaric 8-OH, 9c12c-18:2; Lanoceric di-OH 30:0; Lamenallenic 5c,6c-18:2; Lactobacillic 11,12-Mt 18:0; Lauric 12:0; Lauroleic 9c-12:1; Lesquerolic 14-OH 11c-20:1; Leucic 2-OH, 4-Me 5:0; Levulinic 4-oxo-5:0; Licanic (α) 4-oxo 9c11t13t-18:3; Licanic (β) 4-oxo 9t11t13t-18:3 (Trans-form of α-licanic acid); Lignoceric 24:0; Linderic 4c-12:1; Linelaidic 9t12t-18:2; Linoleic 9c12c-18:2; Linolenelaidic 9t12t15t-18:3; Linolenic 9c12c15c-18:3; Lumequic 21c-30:1; Linusic 9,10,12,13,15,16-hexaOH 18:0; Lycaonic 12-oxo-18:0; Lycopodic 11t-16:1; Maleic 2c-4:1 di-acid; DL-Malic 2-OH 4:0 di-acid; Malonic 3:0 di-acid; Malvalic 8,9-P 8c-18:1; Malyngic 9S,12R,13S tri-OH 10t,15c-18:2; Manaoic/manoaic 11-Cp 6c-11:0; Mangold's acid 9t11t-18:2; Margaric 17:0; Matricaria acid 2t4a6a8t-10:4; Z-E- Matricaric 2c4a6a8t-10:4; Mead's acid 5c8c11c-20:3; Megatomic (megatomoic) 3t5c-14:2; Mesaconic (E)-2-Me-4:1 di-acid; Melissic 30:0; Mikusch's acid 10t12t-18:2; Minquartynoic 17-OH-9a11a13a15a-18:4; Montanic 28:0; Mycinonic 4-Me, 5-OH 2t-7:1; Mycoceranic 2,4,6-tri-Me 26:0; Mycolic RCHOHCH(R')COOH; Mycolipenic 2,4,6-tri-Me-2t-24:1; Mycomycin 3t5c7e8e10a12a-13:6; Myristelaidic 9t-14:1 (Trans form of myristoleic acid) Myristic 14:0; Myristoleic 9c-14:1; Nemotinic 4-OH, 5e6e8a10a-11:4; Nerolic 3,7-di-Me 2c6e-8:2; Nervonic 15c-24:1; Nisinic 24:6, all- cis-6,9,12,15,18,21; Norlinoleic 8c11c-17:2; Norlinolenic 8c11c14c-17:3 Libiatae Obtusilic 4c-14:1; Oleic 9c-18:1; Oncobic 15-Cp 8c-15:1; Oxalic 2:0 di-acid; Palmitelaidic 9t-16:1 (trans form of petroselinic acid); Palmitic 16:0; Palmitoleic 9c-16:1; Parinaric (α) 9c11t13t15c-18:4; Parinarium laurinum Parinaric (β) 9t11t13t15t-18:4 (*trans* form of α-parinaric acid); Paullinic 13c-20:1; Pelargonic 9:0; Palmitvaccenic 11c-16:1; Petroselaidic 6t-18:1 (Trans form of petroselinic acid); Petroselinic 6c-18:1; Phellogenic 22:0 di-acid; Phellonic 22-OH 22:0; Phlomic 7e,8e-20:2; Phthioic 3,13,19-tri-Me 23:0; Physeteric 5c-14:1; Phytanic 3,7,11,15-tetra-Me 16:0; Phytenoic 3,7,11,15-tetra-Me 2e-16:1; Pimelic 7:0 di-acid; Pinolenic 5c9c12c-18:3; Pivalic 2,2-di-Me 3:0; Podocarpic 5c11c14c-20:3; Pristanic 2,6,10,14-tetra-Me 15:0; Propioic (propynoic) 2a-3:0; Pseudoeleostearic 10t12t14t-18:3; Psyllic 33:0; Punicic 9c11t13c-18:3; Pyrulic 8a10t-17:2; Rhodinic 3,7-di-Me,6e-8:1; Ricinelaidic 12-OH 9t-18:1 (trans form of ricinoleic acid); Ricinenic 9c,11c-18:2 Ricinoleic 12-OH 9c-18:1; Rosilic 10-OH 18:0; Roughanic acid 16:3(n-3); Rumenic 9c11t-18:2; Sabinic 12-OH 12:0; Sapienic 6c-16:1; Sarcinic 12Me-14:0; Sativic (santivinic) 9,10,12,13-tetraOH 18:0; Scoliodonic 24:5; Sebacic 10:0 di-acid; Sebaleic 5c,8c-18:2; Shibic 26:5; Sorbic 2t4t-6:2 di-acid; Stearic 18:0; Stearidonic 6c9c12c15c-18:4; Stearolic 9a-18:1; Sterculic 9,10-P 9c-18:1; Sterculynic 9,10-Mt 9c17a-18:2; Stillingic 2c4t-10:2; Suberic 8:0 di-acid; Succinic 4:0 di-acid; Tariric 6a-18:1; Tartaric 2,3-diOH 4:0 di-acid; Taxoleic 5c9c-18:2; Thalictric 5t-18:1; Thaspic 16:0 di-acid; (+/-) Threonic 2,3,4 tri-OH 4:0; D-Threonic 2R,3S,4 tri-OH 4:0; L-Threonic 2S,3R,4 tri-OH 4:0; Threonine 2-NH2, 3-OH 4:0; Thynnic 26:6 (n-3); Tiglic (2E)-2-Me 4:1; Traumatic 2t-20:1 di-acid; Tsuduic 4c-14:1; Tsuzuic 4c-14:1; Tuberculostearic 10-Me 18:0; Undecylenic 10e-11:1; Ursolic 30-OH-30:0; Ustilic 15,16-diOH 16:0; Ustilic B 2,15,16-triOH 16:0; Valeric 5:0; Valproic 2-Propyl 5:0; 9,10,12,13-tetraOH-22:0; Vernolic cis-12,13-ep, 9c-18:1; Wyeronic acid 4,7-ep, 8-oxo, 2t9a11c-14:5; Ximenic 17c-26:1; Ximenynic 9a11t-18:2; Ximenynolic 8-OH, 9a11t-18:2; Xionenynic 8a10c-18:2; and any isomers, salts, free salts, derived acids and derives bases thereof, can also be utilized for the construction of the compositions of the present application.

The concentrations in which such at least one cholesterol molecule or any of its derivatives, and/or mono, di or triglyceride molecule can be present in the compositions of the present disclosure can range from about 0.00193949 mM to about 30 mM.

Oxidation protector molecules such as α-tocopherol may also be added to the pharmaceutical compositions of the present application to reduce the oxidation of the phospholipids utilized. These oxidation protectors can thus be selected from a tocopherol molecule, α -tocoferol; β-tocoferol; γ-tocoferol; δ-tocoferol; and any aptamers, isomers, salts, free salts, free bases, free acids, derived acids and derived bases thereof. Also included in the present application are other types of oxidation protectors such as, without limitation to, sulphur compounds; anthocyanins; carotenes (α, β, δ); catechins; copper compounds, cryptoxanthins; flavonoids; indole compounds; isoflavonoids; lignans; lutein; lycopene; manganese compounds; polyphenols, including BHT and BHA; selenium; vitamins (A, C, D, E); zinc compounds.

The concentrations in which such at least one oxidation protector molecules or any of its derivatives can be present in the compositions described in this application can range from about 0.00158663 mM to about 1,14237360 mM., preferably from about 1 mM to about 500 mM, and more preferably from about 1mM to about 10mM.

Water is utilized in the form a solvent and as formulating vehicle for the compositions of the present application. By "water", the present application encompasses any type of water, from distilled, purified, filtered, non-ionic, acid, alkaline, hard (D₂O) and any other variant of water as it fits the requirements of the compositions and processes of the present application. The amount of water utilized to prepare the compositions and present as part of the compositions can range from >0% to <100%.

Saline and non-saline buffers may be also utilized for the preparation of the compositions of the present application in order achieve a specific pH value during preparation or in the resulting products *per se.* The buffers can be utilized as a single buffer or in any mixture of at least two of them, same that can include, without limitation: saline phosphate buffer, sodium acetate/acetic acid, Na₂HPO₄/citric acid, potassium hydrogen phthalate/sodium hydroxide, disodium hydrogen phthalate/sodium dihydrogen orthophosphate, dipotassium hydrogen phthalate/potassium dihydrogen orthophosphate, potassium dihydrogen orthophosphate/sodium hydroxide.

The concentrations of these saline and/or non-saline buffers at any stage during the preparation or in the final products *per se* can range from about 0.1 mM to about 10 mM

Organic Solvents are also utilized in the preparation of the compositions on the present application, and can be selected from the group comprising: acetic acid, acetone, acetonitrile, acetyl acetone, 2-aminoethanol, aniline, anisole, benzene, benzonitrile, benzyl alcohol, 1-butanol, 2-butanol, i-butanol, 2-butanone, t-butyl alcohol, carbon disulfide, carbon tetrachloride, chlorobenzene, chloroform, cyclohexane, cyclohexanol, cyclohexanone, di-n-butylphthalate, 1,1-dichloroethane, 1,2-dichloroethane, diethylamine, diethylene glycol, diglyme, dimethoxyethane (glyme), N,N-dimethylaniline, dimethylformamide (DMF), dimethylphthalate, dimethylsulfoxide (DMSO), dioxane, ethanol, ether, ethyl acetate, ethyl acetoacetate, ethyl benzoate, ethylene glycol , glycerin, heptane, 1-heptanol, hexane, 1-hexanol, methanol, methyl acetate, methyl t-butyl ether (MTBE), methylene chloride, 1-octanol, pentane, 1-pentanol, 2-pentanol, 3-pentanol,. 2-pentanone, 3-pentanone, 1-propanol, 2-propanol, pyridine, tetrahydrofuran (THF), toluene, p-xylene.

The aforementioned solvents can be utilized from about 0.001% to 95% v/v or w/w in the pharmaceutical compositions due to the fact that in the concentrated product the organic solvent used is eliminated, being the upper limit of the concentration utilized for the sphere preparation stage in the lipid flows of the preparation systems.

In some cases, the pharmaceutical compositions of the present application can also include an emulsifier or surfactant agent, which can be anionic, including Soaps and other Carboxylates, products of sulfonation and sulfatation, sulfates, sulfonates; non-ionic, including Ethoxylated Alcohols and Alkylphenols, Fatty acid Esters, and Nitrogenated nonionic Surfactants; cationic, including linear Alkyl-amines and Alkyl-ammoniums and Nitrogenated surfactants with a second hydrophile; or amphoteric in nature. Also included are Silicon Surfactants, Fluorinated Surfactants, Polymeric Surfactants or Surfactant Polymers and Association Polymers. Included in this category are also the lipidic molecules listed above, which can act as emulsifiers/surfactants. Without limitation, surfactants according to the present application can be selected from the group comprising: Polysorbates (Tween™) and any of its derivatives, Sodium dodecyl sulfate (sodium lauryl sulfate) and any of its derivatives, Lauryl dimethyl amine oxide and any of its derivatives, Cetyltrimethylammonium bromide (CTAB) and any of its derivatives, Polyethoxylated alcohols and any of its derivatives, Polyoxyethylene sorbitan and any of its derivatives, Octoxynol and any of its derivatives, N,N-dimethyldodecylamine-N-oxide and any of its derivatives, Hexadecyltrimethylammonium bromide (HTAB) and any of its derivatives, Polyoxyl 10 lauryl ether and any of its derivatives, Bile salts (sodium deoxycholate, sodium cholate) and any of its derivatives, Polyoxyl castor oil and any of its derivatives, Nonylphenol ethoxylate and any of its derivatives, Cyclodextrins and any of its derivatives, Lecithin and any of its derivatives, Methylbenzethonium chloride and any of its derivatives, sorbitan monolaurate (Dodecanoic acid [2-[(2R,3R,4S)-3,4-dihydroxy-2-tetrahydrofuranyl]-2-hydroxyethyl]ester) and any of its derivatives including [2-[(2*R*,3*S*,4*R*)-3,4-dihydroxitetrahydrofuran-2-il]-2-hydroxiethyl]octadecanoate; [2-[(2*R*,3*S*,4*R*)-4-hydroxi-3-octadecanoiloxi-oxolan-2-il]-2-octadecanoiloxiethyl] octadecanoate; de [2-[(2*R*,3*S*,4*R*)-3,4-dihydroxitetrahydrofuran-2-il]-2-hydroxiethyl]dodecanoate; ([2-(3,4-dihydroxioxolan-2-il)-2-hydroxiethyl]-(*E*)-Octadec-9-enoate; [(2R)-2-[(3R,4S)-4-hydroxi-3-[(Z)-octadec-9-enoil]oxioxolan-2-il]-2-[(Z)-octadec-9-enoil]oxiethyl] (Z)-octadec-9-enoate; [2-[(2*R*,3*S*,4*R*)-3,4-dihydroxytetrahydrofuran-2-il]-2-hydroxietthyl]Hexadecanoate; Polyoxyethylene (20) sorbitan monolaurate; 2-[2-[3,4-bis(2-methoxyethoxy)oxolan-2-yl]-2-(2-methoxyethoxy)ethoxy]ethyl hexadecanoate; Polyoxyethylene sorbitan monostearate; Polyoxyethylene sorbitan tristearate; Polyoxyethylene sorbitan monooleate, and any isomers, salts, free salts, derived acids and derives bases thereof.

By "spheres", or "sphere -like" compositions, the present application intends to encompass different types of liposome-like particles which can be, depending on their size; Small Unilamellar Spheres (SUS): from about 40 nm to about ≤ 100 nm; Large Unilamellar Spheres (LUS): from about 100 nm to about ≤ 500 nm; Multilamellar Spheres (MLS): with sizes even larger than about 1 mm (greater than 1000 nm); or any possible combination of the types of sizes described above. The names utilized herein are only illustrative and cannot be considered as an ultimate classification of the particles by its size; as one person skilled in the art would clearly appreciate that the terms "small", "large", "sphere" are relative and can vary depending on the context and desired application of the technology.

In some cases, the size of the sphere particles can be of about ≤ 100 nm for dermal, ophthalmic, or sublingual applications for instance, while for oral administration, sizes between about 150 nm and 450 nm can be used. Again, the names utilized herein are only illustrative and cannot be considered as an ultimate classification of the particles by its application; as one person skilled in the art would clearly appreciate that despite the size of the compositions, therapeutic, preventive and/or adjuvant effects could be achieved regardless of the naming given to the examples of routes of administration given.

Pharmaceutical compositions, as final products, can also be in the form of lyophilized powders or powder-like form containing from 0 to <2% of water content.

The pH of the pharmaceutical compositions described in the present application can range from about 4.0 to about 9.0. In some cases, the compositions can have a pH value of about 6.5.

### Processes for preparing the pharmaceutical compositions.

In the construction of the sphere and or sphere-like pharmaceutical compositions of the present application, microfluidic technology can be used, from which both unilamellar and multilamellar spheres can be obtained. Figure 4 shows a flow chart of one of the examples of the process described in the present application for preparing the compositions (CannSpheres®) of the present application.

The process starts with a precise weighting of the raw material, i.e. isolated cannabinoid compounds or a mixture of at least two cannabinoid compounds, followed by the preparation of the lipid phase and the aqueous phase with their respective ingredients.

In some cases of the process for preparing the compositions of the present application, the aqueous phase can be constructed as follows:
1) Buffer system including at least one buffer or a mixture of at least two buffers
2) Water

In some cases of the process for preparing the compositions of the present application, the lipid phase can be constructed as follows:
1) An isolated Cannabinoid, or a mixture of at least two isolated cannabinoids,
2) A lipid,
3) Oxidation protector,
4) A second aiding lipid
5) Organic Solvent

In some cases of the process for preparing the compositions of the present application, the aqueous phase can be constructed as follows:
1) Buffer system including at least one buffer or a mixture of at least two buffers
2) Water
3) a surfactant agent
4) an emulsified Cannabinoid compound

In a further embodiment of the process for preparing the compositions of the present application, the lipid phase can be constructed as follows:
1) Optionally, an isolated Cannabinoid, or a mixture of at least two isolated cannabinoids,
2) A lipid,
3) Oxidation protector,
4) A second aiding lipid
5) Organic Solvent

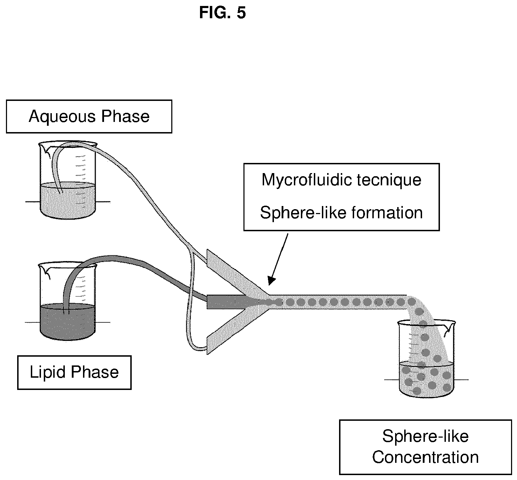

Those two phases are later processed to form the spheres of the present application utilizing different critical parameters of flow rates, temperatures, pH, flow radius, etc. For instance, the Lipid phase flow can range from about 0.01 ml/min - 15.0 ml/min, aqueous phase flow can range from about 0.5 ml/min -15.0 ml/min, the Total flow range can therefore be from about 0.6ml/min to 15.0 ml/min; the flow can vary from 1-20 ml/min, the temperature for the entire process can range from about 0 to about 70 ° C, and the pH value of the different stages of the process from about 4.0 to about 9.0. Figure 5 is a representation of the micro-fluid technique utilized to perform the methods described above, in this particular stage of the process, different examples for the preparation of composition can be achieved by altering the lipid phase flux, the aqueous phase flux, temperature and pH.

Further stages of the process include the concentration of the pharmaceutical composition already in the form of spheres, in which cannabinoid amounts are determined, morphology of the spheres, particle size, Z potential and viscosity are determined. A final step of one of the examples of the process described is marked by the evaporation of the remainder of the organic solvent, where a determination is made to secure a solvent residue level under about 2%.

In another case, and optional final stage of lyophilization is also possible to obtain lyophilized powders or powder-like form containing from 0 to <2% of water content.

The values of operation of the methods of the present application are shown below:

### Lipid flow values:

0.01 to 15.0 ml/min, 0.1 to 10.0 ml/min, or 0.3 to 9.0 ml/min.

### Flow values for the aqueous phase:

0.5 to 15.0 ml/min; from 1.0 to 12.0 ml/min; or from 3.0 -10.0 ml/min

### Total flow: from 0.6 to 15.0 ml/min / Flow Radius: 1-20 ml/min

### Temperature of the process: 0-70°C

For instance, four examples of the process of the present application can have the following values:

| **Lipid flow (ml/min)** | **Buffer flow (ml/min)** | **Total flow=Lipid flow + Buffer flow (ml/min)** | **Flow radius=Buffer flow/Lipid flow** |
|---|---|---|---|
| **2** | **2** | **4** | **1** |
| **0.5** | **3.5** | **4** | **7** |
| **1** | **11** | **12** | **11** |
| **0.5** | **5.5** | **6** | **11** |

The processes of preparation for the compositions of the present application allows therefore to obtain adequate size particles to secure an appropriate bioavailability of cannabinoid compounds by selecting very specific values in their critical parameters which at the same time allows for a precise metered dose of isolated cannabinoid compounds or mixtures of at least two cannabinoid compounds. The effectivity in securing the different sizes of the individual compositions of the present application and the encapsulation rates of the process, guarantees that a pharmaceutical product will contain the desired and specific doses of cannabinoids, as the dose of cannabinoid either inside each sphere and/or inside a final product containing the compositions can be known for certain.

Also, the typical flaws art associated with the uncertainty of the effective amount of cannabinoids that are encapsulated or otherwise associated to the systems described therein are avoided with the processes of the present application as it was surprisingly discovered that utilizing the critical values of flow, radius and temperature results in encapsulation of isolated or mixtures of cannabinoids in an almost 100% rate; a later particle size distribution having at least less than 5% in its variation coefficient; which allows the selection and using of specific particle sizes that will provide the therapeutic effect and for the benefits already described above.

### Examples.

The following examples are non-limitative illustrative of some of the examples described in the present application. However, the skilled artisan, based on all the technical information provided before can indeed be able to understand and construct the rest of the examples included within the scope of the present application.

### Example 1: Preparations made within the scope of the parameters are showed into the following table 1:

**Table 1. Examples of preparations covering the general scope of the application**

| **CELL type H/droplet junction chip 190** | **A (aqueous phase)** | **B (Lipid phase)** | **Phospholipid flux (ml/min)** | **Buffer flux (ml/min)** | **Total flux (ml/min)** | **Flux Radius** | **Phospolipid concentration (mg/ml)** | **Cannabinoid concentration (mg/ml)** |
|---|---|---|---|---|---|---|---|---|
| TEST 1 | CITRATE | phosphatidylcholine = 20 mg/ml CBD=6.66 mg/ml Etanol cbp 1 ml | 1 | 3 | 4 | 3.00 | 5.00 | 1.67 |
| TEST 2 | CITRATE | | 0.5 | 10 | 10.5 | 20.00 | 0.95 | 0.32 |
| TEST 3 | PHOSPATE | phosphatidylcholine = 20 mg/ml CBD=6.66 mg/ml Ethanol cbp 1 ml | 1 | 9 | 10 | 9.00 | 2.00 | 0.67 |
| TEST 4 | PHOSPATE | phosphatidylcholine = 60 mg/ml CBD=20 mg/ml Ethanol cbp 1 ml | 1 | 9 | 10 | 9.00 | 6.00 | 2.00 |
| TEST 5 | CITRATE | phosphatidylcholine = 60 mg/ml CBD=20 mg/ml Ethanol cbp 1 ml | 6 | 10 | 16 | 1.67 | 22.50 | 7.50 |
| TEST 6 | CITRATE | phosphatidylcholine =150 mg/ml CBD=50 mg/ml Ethanol cbp 1 ml | 1 | 9 | 10 | 9.00 | 15.00 | 5.00 |
| TEST 7 | PHOSPATE | phosphatidylcholine = 150 mg/ml CBD= 50 mg/ml Ethanol cbp 1 ml | 1 | 9 | 10 | 9.00 | 15.00 | 5.00 |
| TEST 8 | PHOSPATE | Hydrogenphosphatidy Icholine = 450 mg/m= 20 mg/ml CBD= 6.66 mg/ml Ethanol cbp 1 ml | 1 | 9 | 10 | 9.00 | 15.00 | 0.67 |
| TEST 9 | PHOSPATE | Hydrogenphosphatidy Icholine = 450 mg/ml CBD=150 mg/ml Ethanol cbp 1 ml | 0.3 | 3 | 3.3 | 10.00 | 40.91 | 13.64 |

Regarding the parameters that were evaluated; combinations of column flows (A and B) were made, which contained phospholipids and buffers at different concentrations. As a result, different sizes and distributions of dispersed mono particles were obtained, confirming the efficiency of the parameters utilized for encapsulating the at least one cannabinoid compound.

### Example 2: Representative examples of particular cases of the pharmaceutical compositions (as prepared by the process described in the present application):

### Example 2.a

| **Aqueous phase** | |
|---|---|
| **Component (at least one)** | **Concentration** |
| Sodium Chloride | 0.0137 - 0.5 M |
| Potassium Chloride | 0.0003 - 0.005 M |
| Di-basic sodium phosphate | 0.001 - 0.05 M |
| Mono-basic potassium phosphate | 0.00002 - 0.003 M |
| Purified water | To complete 100% |

| **Lipid Phase** | |
|---|---|
| **Component (at least one)** | **Concentration** |
| Cannabinoid (CBD, THC, or any other(s)) | 0.0079504 mM to 1.9080935 mM. |
| L-α-phosphatidylcholine (soybean) | 0.0079504 mM to 19.0809350 mM |
| N-(carbonyl-methoxypolyethylene glycol-2000)-1,2-distearoyl-sn-glycero-3-phosphoethanolamine, Sodium Salt | 0.0007959 mM to 9.5404675 mM |
| Cholesterol | 0.0007959 mM to 5.7242805 mM |
| α-tocopherol acetate | 0.0007959 mM to 3.8161870 mM |
| Organic Solvent cbp | To complete 100% |

| **Resulting composition** | |
|---|---|
| **Component** | **Concentration** |
| Cannabinoid (CBD, THC, or any other(s)) | 0.0079504 mM to 1.9080935 mM. |
| L-α-phosphatidylcholine (soybean) | 0.0079504 mM to 19.0809350 mM |
| N-(carbonyl-methoxypolyethylene glycol-2000)-1,2-distearoyl-sn-glycero-3-phosphoethanolamine, Sodium Salt | 0.0007959 mM to 9.5404675 mM |
| Cholesterol | 0.0007959 mM to 5.7242805 mM |
| α-tocopherol acetate | 0.0007959 mM to 3.8161870 mM |
| Sodium Chloride | 0.137 M |
| Potassium Chloride | 0.0027 M |
| Di-basic sodium phosphate | 0.01 M |
| Mono-basic potassium phosphate | 0.0018 M |
| Water | Remaining up to 100% |

### Example 2.b

| **Aqueous phase** | |
|---|---|
| **Component (at least one)** | **Concentration** |
| Sodium Chloride | 0.0137 - 0.5 M |
| Potassium Chloride | 0.0003 - 0.005 M |
| Di-basic sodium phosphate | 0.001 - 0.05 M |
| Mono-basic potassium phosphate | 0.00002 - 0.003 M |
| Purified water | To complete 100% |

| **Resulting composition** | |
|---|---|
| **Component** | **Concentration** |
| Cannabinoid (CBD, THC, or any other(s)) | 7 mM to 13 mM |
| L-α-phosphatidylcholine (soybean) | 65 mM to 93 mM |
| N-(carbonyl-methoxypolyethylene glycol-2000)-1,2-distearoyl-sn-glycero-3-phosphoethanolamine, Sodium Salt | 0.0007959 mM to 9.5404675 mM |
| Cholesterol | 10 mM to 15 mM |
| α-tocopherol acetate | 0.0007959 mM to 3.8161870 mM |
| Sodium Chloride | 0.137 M |
| Potassium Chloride | 0.0027 M |
| Di-basic sodium phosphate | 0.01 M |
| Mono-basic potassium phosphate | 0.0018 M |
| Water | Remaining up to 100% |

| **Resulting composition** | |
|---|---|
| **Component** | **Concentration** |
| Cannabinoid (CBD, THC, or any other(s)) | 7 mM to 13 mM. |
| L-α-phosphatidylcholine (soybean) | 65 mM to 93 mM |
| N-(carbonyl-methoxypolyethylene glycol-2000)-1,2-distearoyl-sn-glycero-3-phosphoethanolamine, Sodium Salt | 0.0007959 mM to 9.5404675 mM |
| Cholesterol | 10 mM to 15 mM |
| α-tocopherol acetate | 0.0007959 mM to 3.8161870 mM |
| Sodium Chloride | 0.137 M |
| Potassium Chloride | 0.0027 M |
| Di-basic sodium phosphate | 0.01 M |
| Mono-basic potassium phosphate | 0.0018 M |
| Water | Remaining up to 100% |

### Example 2.c

| **COMPONENTS** | **UNITARY FORMULA** | | **% W/V** | **FUNCTION** |
|---|---|---|---|---|
| **CBD** | **25.230** | **Mg** | **2.52** | **Active Ingredient** |
| **Phosphatidylcholine** | **70.000** | **Mg** | **7.00** | **Solubilizer** |
| **PEG-Phoshpatidylcholine** | **3.010** | **Mg** | **0.30** | **Stabilizer** |
| **Cholesterol** | **5.300** | **Mg** | **0.53** | **Stabilizer** |
| **BHT** | **0.500** | **Mg** | **0.05** | **Antioxidant** |
| **BHA** | **0.500** | **Mg** | **0.05** | **Antioxidant** |
| **Sodium deoxycholate** | **8.000** | **Mg** | **0.80** | **Surfactant** |
| **Ethanol 96% q.s.** | **1.000** | **MI** | **-** | **Vehicle** |
| **Phpsphate buffer 10 mM q.s.** | **10 ml** | **MI** | **-** | **Vehicle** |

The process for making the formulation of example 2.c is as follows:
1. Preparation of the lipid phase and the aqueous phase with their respective ingredients.
2. Liposome formation occurs by the microfluidic technique using a 5-channel microfluidic hydrodynamic focusing (MHF) chip using a total flow (TFR) of 8 to 12 ml/min and a flow range (FRR) of 8 to 50, at a temperature of 25°C. The pH value throughout the process is around 7.
3. The resulting suspension obtains a concentration of 0.5 to 2.5 mg/ml of CBD depending on the flow conditions with a particle size of 319.8 nm with a polydispersion index of 13.5%. The characterization is carried out by determining the particle size distribution by means of Dynamic Light Scattering, as well as zeta potential. The concentration is determined by means of HPLC.
4.- Optionally, the suspension is lyophilized, adding a cryoprotectant and freezing the by means of a cooling ramp at 4°C for 1 h, then at -20°C for 1 h, and finally at -80°C for 3 h. After freezing, the samples were placed in the lyophilizer (LABCONCO FreeZone 1) under the following conditions: -50°C and pressure of 0.142 mBar for 24 h.

### Example 3: lyophilization of the liposome suspension

Tests were carried out to identify the critical parameters of process control, and the appropriate concentration of the cryoprotectant was evaluated to favor the obtaining of a lyophilizate that meets the established quality attributes.

The systems used were prepared using a concentration of Phosphatidylcholine (FC) of 70 mg/ml and a concentration of Phosphatidylcholine-PEG (FC-PEG) of 3.0 mg/ml in the lipid solution. As a continuous phase, phosphate buffer pH 6.5 10 mM was used.

Once the systems were prepared, the effect of the cryoprotectant (mannitol) was evaluated in concentrations of 1, 3 and 5% w/v, dissolving the required amount of the aforementioned components in the suspension obtained, the above with the help of constant magnetic stirring until complete dissolution. Likewise, the systems were evaluated without the addition of a cryoprotectant. Subsequently, 5 ml of each suspension were placed in 20 ml vials, the above in duplicate.

For the freezing stage two conditions were evaluated:
For the first case, the samples were frozen directly by placing them in an ultra-freezer at - 80°C for 3 hours.

For the second case, a freezing ramp was used, initially cooling the samples to 4°C for 1 h, later freezing them at -20°C for 1 h, and finally to -80°C for 3 h.

After the freezing process, the samples were placed in the freeze dryer under the following parameters:
- Condenser temperature: -50°C
- Pressure: 0.142 Bar
- Time: 24 h

Table 2 shows the experimental matrix for the evaluation of cryoprotectants.

**Table 2 Experimental matrix for the evaluation of cryoprotectant concentration and lyophilizate conditions.**

| **TEST** | **Cryoprotectant concentration (% w/v)** | **Freezing ramp** |
|---|---|---|
| 1 | 1 | Yes |
| 2 | 1 | Yes |
| 3 | 3 | Yes |
| 4 | 3 | Yes |
| 5 | 5 | Yes |
| 6 | 5 | Yes |
| 7 | 1 | No |
| 8 | 1 | No |
| 9 | 3 | No |
| 10 | 3 | No |
| 11 | 5 | No |
| 12 | 5 | No |
| 13 | 0 | Yes |
| 14 | 0 | Yes |
| 15 | 0 | No |
| 16 | 0 | No |

Once the lyophilization time elapsed, the samples were sheltered at 4°C, protected from light until used.

To evaluate the stability of the systems after freezing and lyophilization, the samples obtained were resuspended with 5 ml of 10 mM pH 6.5 phosphate buffer and shaken until obtaining a homogeneous suspension. Particle size distribution and polydispersity index were analyzed in the Litesizer equipment.

### Example 4: In vitro bioavailability and cell cytotoxicity test

The viability and cellular cytotoxicity were evaluated by XTT (tetrazolium chloride) colorimetric reduction assay, which is reduced to formazan products by the action of NAPH and coenzyme Q present in metabolically active cells. For this test, the XTT reagent is incubated for 4 hours in cells previously exposed to a treatment (2x10⁴ cells / well) and the formazan products are quantified by absorbance on an Epoch spectrophotometer.

In order to quantify the bioavailability by the cellular uptake of the spheres, the intracellular quantification of CBD was performed, using a culture of intestine cells (Hs 1.Int (ATCC® CRL-7820 ™ )). They were cultured according to the recommendations of ATCC 80% in Dulbecco's Modified Eagle Medium (DMEM) complete, with 5% fetal bovine serum (FBS) at 37°C/5% CO₂.

Cells were transferred to 24-well plates at a density of 1 x 10⁵ cells per well (0.5 ml), in triplicate, in the presence of culture medium (preheated DMEM, supplemented with 10% FBS; Sigma-Aldrich, UK) and increasing concentrations of CBD-spheres, homogenized and incubated for 24 hours. Subsequently, the contents of the well are centrifuged (1000 G-15 min), the supernatant is recovered, obtaining the liposomal CBD-unbound to the cells, an organic phase extraction (DCM) is performed and subsequently analyzed (HPLC, UV- Vis) by comparing against a standard CBD curve to obtain concentration and the % uptake is obtained with respect to the added concentration (indirect method).

The cell button is washed with 1 ml of PBS and then incubated twice with 0.5 ml of glycine-HCl (50 mM, pH 2.8). The supernatant contains the fraction of CBD-liposomal attached or bound to the cell membrane, an organic phase extraction (DCM) is performed and subsequently analyzed (HPLC, UV-Vis) comparing against the standard CBD curve to obtain concentration and get % membrane bound.

The cell concentrates are washed with 1 ml of PBS and twice with 0.5 ml of 1.0 M NaOH (cell lysis) to recover the Interiorized in the cytoplasm, the samples are centrifuged 200 g for 5 min to remove the cell debris , to the supernatant an organic phase extraction (DCM) is performed and subsequently analyzed (HPLC, UV-Vis) comparing against standard CBD curve to obtain concentration and % cell uptake is obtained (direct method). The detection was accompanied by a mass spectrometry analysis (TQD MS/MS).

### Example 4: Effect the CBDs and THCs sphere systems as therapeutic agents on gastrointestinal cancer.

32 patients suffering from different stages of gastrointestinal cancers were administered with the pharmaceutical compositions of the present application, resulting in at least 10% of improved tumoral reduction response in comparison with chemotherapy alone.

### Example 5: Effect the CBDs and THCs sphere systems as therapeutic agents on Irritable Bowel Syndrome.

50 patients were administered with the pharmaceutical composition systems of the present application, showing significant improvements on the inflammatory pattern, oxidative stress and plasmatic cytosine levels of their IBS.

## Claims

1. A cannabinoid-containing pharmaceutical composition comprising;
i) a pharmaceutically acceptable polymer coating;
ii) at least one lipid component;
iii) a lipid-aqueous interface, and
iv) a nucleus,
wherein the composition incorporates or otherwise carries a cannabinoid or a mixture of cannabinoids.

2. The cannabinoid-containing pharmaceutical composition according to claim 1, further comprising, in its outermost layer, a Fab (antigen-binding fragment) having absorbed or adsorbed or secured to a selective targeting molecule, optionally wherein the selective targeting molecule incorporates or otherwise carries the cannabinoid or the mixture of cannabinoids, or another cannabinoid-receptor agonist compound.

3. The cannabinoid-containing pharmaceutical composition according to claim 1, wherein the pharmaceutically acceptable polymer coating incorporates or otherwise carries the cannabinoid or the mixture of cannabinoids.

4. The cannabinoid-containing pharmaceutical composition according to claim 1, wherein the at least one lipid component is in the form of a single or multiple membrane, optionally wherein the at least one lipid component is in the form of a single membrane.

5. The cannabinoid-containing pharmaceutical composition according to claim 4, wherein the single or multiple membrane incorporates or otherwise carries the cannabinoid or the mixture of cannabinoids.

6. The cannabinoid-containing pharmaceutical composition according to claim 1, wherein the lipid-aqueous interface incorporates or otherwise carries the cannabinoid or the mixture of cannabinoids.

7. The cannabinoid-containing pharmaceutical composition according to claim 1, wherein the nucleus contains the cannabinoid compound or the mixture of cannabinoid compounds.

8. The cannabinoid-containing pharmaceutical composition according to claim 1, wherein the aqueous interphase comprises the cannabinoid and at least one solvent, at least one buffer, and water.

9. The cannabinoid-containing pharmaceutical composition according to claim 1, further comprising at least one oxidation protector.

10. A process for making the cannabinoid-containing pharmaceutical composition according to claim 1, comprising:
a) preparing an aqueous phase of the composition;
b) preparing a lipid phase of the composition;
c) processing the resulting phases of steps a) and b), using critical parameters of flow rates, temperatures, pH, and flow radius by microfluidic technology.

11. The process according to claim 10, wherein the lipid phase flow ranges from about 0.01 ml/min - 15.0 ml/min, the aqueous phase flow ranges from about 0.5 ml/min - 15.0 ml/min, the total flow ranges from about 0.6 ml/min to 15.0 ml/min, the flow radius ranges from 1-20 ml/min, the temperature for the entire process ranges from about 0 to about 70°C, and the pH value of each stage of the process ranges from about 4.0 to about 9.0.

12. The process according to claim 10, further comprising, lyophilizing the cannabinoid-containing pharmaceutical composition.

13. The cannabinoid-containing pharmaceutical composition according to claim 1, for use in treating, preventing or adjuvating treatments for endocannabinoid pathway-involving conditions in a subject in need thereof.

14. The cannabinoid-containing pharmaceutical composition for use according to claim 13, wherein the endocannabinoid pathway-involving conditions are selected from the group consisting of muscle pain for athletes, cancer, and irritable bowel disease.

15. The cannabinoid-containing pharmaceutical composition for use according to claim 13, wherein the cannabinoid-containing pharmaceutical composition is adapted to a pharmaceutical form suitable to enter a route selected from the group consisting of dermal, ophthalmic, sublingual and oral.
